(19) Europäisches Patentamt

European Patent Office

Office européen des brevets

(11) **EP 3 626 338 A1**

(12) # EUROPEAN PATENT APPLICATION
published in accordance with Art. 153(4) EPC

(43) Date of publication:
**25.03.2020 Bulletin 2020/13**

(21) Application number: **18801959.0**

(22) Date of filing: **09.04.2018**

(51) Int Cl.:
*B01J 23/887* (2006.01)    *B01J 37/00* (2006.01)
*C07C 253/26* (2006.01)    *C07C 255/08* (2006.01)
*C07B 61/00* (2006.01)

(86) International application number:
**PCT/JP2018/014907**

(87) International publication number:
**WO 2018/211858 (22.11.2018 Gazette 2018/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.05.2017 JP 2017100007**

(71) Applicant: **Asahi Kasei Kabushiki Kaisha
Tokyo 100-0006 (JP)**

(72) Inventors:
• **TOMODA, Atsushi**
  **Tokyo 101-8101 (JP)**
• **FUKUZAWA, Akiyoshi**
  **Tokyo 101-8101 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **CATALYST FOR AMMOXIDATION, METHOD FOR PRODUCING SAME AND METHOD FOR
PRODUCING ACRYLONITRILE**

(57)    The catalyst for ammoxidation of the present invention contains a catalyst particle containing molybdenum, bismuth and iron, and has a ratio of hollow particles of 23% or less. Furthermore, a method for producing the catalyst for ammoxidation includes a step of preparing a catalyst precursor slurry containing molybdenum, bismuth and iron and having a solid concentration of 30% by mass or less, a step of spray-drying the catalyst precursor slurry at a drier inlet temperature of 120°C to 240°C to thereby obtain a dried particle and a step of calcining the dried particle at 500 to 750°C.

**EP 3 626 338 A1**

## Description

Technical Field

The present invention relates to a catalyst for ammoxidation, a method for producing the same and a method for producing acrylonitrile.

Background Art

A reaction for producing acrylonitrile by reacting propylene with ammonia to produce acrylonitrile in the presence of molecular oxygen is known as "ammoxidation reaction." This reaction is currently used as a main method for industrially producing acrylonitrile. The ammoxidation reaction may also be used as a reaction for producing methacrylonitrile by reacting isobutene and ammonia.

Catalysts are used in the ammoxidation reaction in order to achieve a good yield of acrylonitrile. For example, a large number of catalysts made of particles containing a composite metal oxide containing Mo-Bi-Fe or Fe-Sb as essential components have been proposed and industrially used. To use this catalyst in a fluidized bed reactor for a long time in a stable manner, not only the size distribution and the shape of catalyst particles need to be suitable for fluidization, but also catalyst particles need to have a high wear resistance and compressive strength. Thus, many catalysts prepared by making the above composite metal oxide supported on a carrier such as silica, alumina, zirconia and titania have been proposed.

Patent Literature 1 discloses a catalyst made of particles containing a Mo-Bi-Fe-based composite metal oxide and a silica carrier. Patent Literature 1 discloses that the shape of the resulting catalyst was observed by a scanning electron microscope and the result was that a solid spherical powder was obtained in most of the examples and comparative examples, while the powder of comparative example 8, in which the content of the carrier was 80% by mass, was a mixture of solid spheres, spherical bodies with holes and distorted spherical bodies with some dents on the surface.

Meanwhile, Patent Literature 2 discloses a catalyst composed of particles containing zeolite and silica, which is used for the reaction to convert hydrocarbon and/or alcohol into propylene. Patent Literature 2 discloses that when the proportion of the void area in a cross-section of the catalyst particle is 30% or less relative to the cross-sectional area of the catalyst particle, the catalyst has excellent mechanical strength. Furthermore, FIGs. 10 to 17 of Patent Literature 2 show a scanning electron micrograph of the shape of the catalyst particle of the comparative examples and a cross-section thereof; the figures show that particles with abnormalities on the surface, such as pore openings and lines, have a large void even in the cross-section.

Although Patent Literature 1 does not disclose observation of cross-sections, it is likely that particles other than those with abnormalities on the surface, such as pore openings and lines, are solid spheres.

Citation List

Patent Literature

Patent Literature 1: Japanese Patent No. 5491037
Patent Literature 2: International Publication No. WO2010/016338

Summary of Invention

Technical Problem

Mechanical strength of catalyst particles is corelated with the amount of carrier, the shape of particles and the presence of voids in the particle. For the amount of carrier, for example, while the attrition strength is 0.8 to 1.6% in the examples and comparative examples of Patent Literature 1, in which the content of a silica carrier is 40% by mass, the catalyst has a higher wear resistance with an attrition strength of 0.2 to 0.5% in the examples and comparative examples, in which the content of the silica carrier is 50% by mass. Meanwhile, the larger the content of carrier, the more the yield of acrylonitrile with a catalyst tends to be reduced. It is therefore preferable to improve attrition strength without using silica excessively or without using silica as a carrier. It is particularly preferable to improve attrition strength by improving the shape of particles.

The present inventors have conducted studies and as a result have found that catalyst particles prepared according to the method disclosed in Patent Literature 1 substantially did not contain particles with abnormalities on the surface, such as pore openings and lines as shown in FIGs. 10 to 17 of Patent Literature 2, but the catalyst particles contained more than 24% of hollow particles having small voids when the cross-section of the particle was observed. Thus, it is assumed that there is still room for improving the technique disclosed in Patent Literature 1 from the viewpoint of wear resistance strength and compressive strength sufficient for withstanding long-term use as a fluidized bed catalyst.

As described above, an ammoxidation catalyst, which has a wear resistance strength and a compressive strength sufficient for withstanding long-term use in a fluidized bed reactor and maintains high yield of acrylonitrile, has not been obtained.

The present invention has been made in view of the above problem, and an object of the present invention is to provide a catalyst for ammoxidation as a fluidized bed catalyst, which has a wear resistance strength and compressive strength sufficient for withstanding long-term use and maintains high yield of acrylonitrile, a method for producing the catalyst and a method for producing acrylonitrile using the catalyst.

Solution to Problem

The present inventors have conducted studies to solve the above problem, and as a result have found that excellent wear resistance strength and compressive strength can be achieved by setting the ratio of hollow particles to a specific range or lower, taking into account even hollow particles without abnormalities on the surface of catalyst particles. The present inventors have also found that a catalyst having the above ratio of hollow particles can be produced by setting the solid concentration of a catalyst precursor slurry and the temperature of spray-drying to a specific range, respectively, and have completed the present invention.

That is, the present invention is as follows.

[1] A catalyst for ammoxidation, comprising a catalyst particle comprising molybdenum, bismuth and iron, wherein the catalyst has a ratio of hollow particles of 23% or less.

[2] The catalyst for ammoxidation according to [1], wherein the catalyst particle comprises a composite metal oxide having a composition represented by the following formula (1):

$$Mo_{12}Bi_aFe_bX_cY_dZ_eO_f \qquad (1)$$

wherein X represents at least one element selected from the group consisting of nickel, cobalt, magnesium, calcium, zinc, strontium, and barium; Y represents at least one element selected from the group consisting of cerium, chromium, lanthanum, neodymium, yttrium, praseodymium, samarium, aluminum, gallium, and indium; Z represents at least one element selected from the group consisting of potassium, rubidium; and cesium, a, b, c, d, e, and f represent an atomic ratio of each element and satisfy $0.1 \leq a \leq 2.0$, $0.1 \leq b \leq 3.0$, $0.1 \leq c \leq 10.0$, $0.1 \leq d \leq 3.0$, and $0.01 \leq e \leq 2.0$, respectively, wherein f represents a number of oxygen atoms needed to satisfy atomic valence requirements of the other existing elements.

[3] A method for producing the catalyst for ammoxidation according to [1] or [2], the method comprising:

a step of preparing a catalyst precursor slurry which comprises molybdenum, bismuth and iron and which has a solid concentration of 30% by mass or less,
a step of spray-drying the catalyst precursor slurry at a drier inlet temperature of 120°C to 240°C to thereby obtain a dried particle, and
a step of calcining the dried particle at 500 to 750°C.

[4] The method for producing the catalyst for ammoxidation according to [3], wherein the catalyst precursor slurry has a solid concentration of more than 3% by mass and 30% by mass or less.

[5] The method for producing the catalyst for ammoxidation according to [3] or [4], wherein a spray drier having an inner diameter of 1,000 mm or more is used.

[6] A method for producing acrylonitrile, the method comprising a step of reacting propylene, molecular oxygen and ammonia in a presence of the catalyst for ammoxidation according to [1] or [2].

Advantageous Effects of Invention

The present invention can provide a catalyst for ammoxidation as a fluidized bed catalyst, which has a high wear resistance strength and compressive strength sufficient for withstanding long-term use and maintains a high yield of acrylonitrile, a method for producing the catalyst and a method for producing acrylonitrile.

Description of Embodiments

In the following, an embodiment for carrying out the present invention (hereinafter simply referred to as "present embodiment") will be described. The present invention is not limited to the following embodiment, and various modification may be made without departing from the spirit of the present invention.

[Catalyst for ammoxidation]

The catalyst for ammoxidation of the present embodiment comprises a catalyst particle comprising molybdenum, bismuth and iron, and the catalyst has a ratio of hollow particles of 23% or less. A catalyst for ammoxidation, in which the ratio of hollow particles is adjusted to 23% or less, has a high wear resistance strength and compressive strength sufficient for withstanding long-term use and maintains high yield of acrylonitrile, as a fluidized bed catalyst.

The above ratio of hollow particles may be adjusted to 23% or less by, for example, using preferred conditions for producing a catalyst for ammoxidation described later, which is not limited to the following.

Here, the hollow particle refers to a particle in which the area occupied by voids is 1% or more based on the area of a cross-section when the cross-section of the particle is observed. Furthermore, a solid particle refers to a particle in which the above area occupied by voids is less than 1% or a particle without voids.

In this regard, the ratio of hollow particles refers to a value obtained by dividing the number of hollow particles observed by the total number of the particles observed and multiplying the resultant by 100. The method of measuring the ratio of hollow particles will be described in Examples described later.

In the present embodiment, the above ratio of hollow particles is preferably 20% or less, and more preferably 18% or less, from the viewpoint of exhibiting a better wear resistance strength and compressive strength.

The lower limit of the ratio of hollow particles is not particularly limited, and is preferably more than 0%, more preferably 1% or more, and further preferably 2% or more, from the viewpoint of productivity.

The catalyst for ammoxidation of the present embodiment has a low ratio of hollow particles and exhibits high wear resistance strength and compressive strength.

Hereinafter the constitution of the catalyst for ammoxidation of the present embodiment will be described in more detail.

(Composition)

The catalyst particle according to the present embodiment contains a composite metal oxide, or the composite metal oxide and a carrier.

The composite metal oxide contains molybdenum, bismuth and iron as essential components. Molybdenum serves as a site of adsorption of propylene and a site of activation of ammonia. Meanwhile, bismuth has a role of activating propylene and abstracting $\alpha$ hydrogen to generate a $\pi$ allyl species. Furthermore, iron has a role of supplying oxygen existing in a gas phase to a catalytically active site through trivalent/divalent redox.

Examples of optional components, which may be contained in the composite metal oxide in addition to the above components, include, but are not particularly limited to: at least one element X selected from the group consisting of nickel, cobalt, magnesium, calcium, zinc, strontium, and barium; at least one element Y selected from the group consisting of cerium, chromium, lanthanum, neodymium, yttrium, praseodymium, samarium, aluminum, gallium, and indium; and at least one element Z selected from the group consisting of potassium, rubidium, and cesium. Herein, the element X forms a molybdate having a moderate amount of lattice defects and has a role of making the transfer of oxygen in a bulk smooth. The element Y may have a redox function in catalyst as well as iron is. The element Z may have a role of suppressing decomposition reaction of main products and starting materials by blocking acid centers existing on the surface of catalyst.

The composite metal oxide preferably has a composition represented by the following formula (1). With such a composition, acrylonitrile selectivity tends to be further improved when the composite metal oxide is used as a catalyst for ammoxidation reaction.

$$Mo_{12}Bi_aFe_bX_cY_dZ_eO_f \qquad (1)$$

wherein X represents at least one element selected from the group consisting of nickel, cobalt, magnesium, calcium, zinc, strontium, and barium; Y represents at least one element selected from the group consisting of cerium, chromium, lanthanum, neodymium, yttrium, praseodymium, samarium, aluminum, gallium, and indium; Z represents at least one element selected from the group consisting of potassium, rubidium, and cesium; a, b, c, d, e, and f represent an atomic ratio of each element and satisfy $0.1 \leq a \leq 2.0$, $0.1 \leq b \leq 3.0$, $0.1 \leq c \leq 10.0$, $0.1 \leq d \leq 3.0$, and $0.01 \leq e \leq 2.0$, respectively, wherein f represents a number of oxygen atoms needed to satisfy atomic valence requirements of the other existing elements.

In the above formula (1), a is preferably $0.15 \leq a \leq 1.0$, and more preferably $0.2 \leq a \leq 0.7$,

in the above formula (1), b is preferably $0.5 \leq b \leq 2.5$, and more preferably $1.0 \leq b \leq 2.0$,

in the above formula (1), c is preferably $3.0 \leq c \leq 9.0$, and more preferably $5.0 \leq c \leq 8.5$,

in the above formula (1), d is preferably $0.2 \leq d \leq 2.0$, and more preferably $0.3 \leq d \leq 1.5$, and

in the above formula (1), e is preferably $0.05 \leq e \leq 1.0$.

When acrylonitrile is industrially produced, usually, a fluidized bed reaction in which a catalyst is fluidized by a reaction gas is selected. Therefore, the catalyst for ammoxidation preferably has a predetermined or higher strength. From such a viewpoint, the catalyst for ammoxidation may be a composite metal oxide supported by a carrier. Examples of carriers for catalyst for ammoxidation include oxides such as silica, alumina, titania and zirconia. Of them, silica, which causes a small reduction in the acrylonitrile selectivity and can significantly improve wear resistance and strength of particles of catalyst, is suitable as a carrier.

The content of the carrier is preferably 30 to 70% by mass, more preferably 35 to 65% by mass, based on the mass of the catalyst for ammoxidation (the total mass of the composite metal oxide and the carrier). When the content of the carrier is 30% by mass or more, the wear resistance and the compressive strength of the catalyst tends to be further improved. Furthermore, when the content of the carrier is 70% by mass or less, the acrylonitrile selectivity tends to be further improved.

Starting materials for silica to be used as a carrier are not particularly limited, and silica sol is preferred. The primary particle diameter of silica contained in silica sol is not particularly limited, and different types of silica each having a different primary particle diameter may be mixed and used.

[Method for producing catalyst for ammoxidation]

The method for producing a catalyst for ammoxidation of the present embodiment comprises a step of preparing a precursor slurry (step (i)), in which a precursor slurry comprising molybdenum, bismuth, iron and silica is prepared, a step of drying (step (ii)), in which the precursor slurry is spray-dried to obtain a dried particle, and a step of calcination (step (iii)), in which the dried particle is calcined.

[Step (i): Step of preparing precursor slurry]

Step (i) is a step of preparing a catalyst precursor slurry (hereinafter also simply referred to as a "precursor slurry") containing molybdenum, bismuth, iron and silica. At that stage, water and carboxylic acid may also be mixed where necessary. In step (i), a precursor slurry may be prepared according to a process for mixing a solution or a slurry containing at least molybdenum and silica with a solution or a slurry containing at least bismuth and iron.

In step (i), the precursor slurry has a solid concentration of preferably 30% by mass or less. When the solid concentration is 30% by mass or less, it is likely that the temperature of the surface of droplets sprayed in the spray-drying of step (ii) described later is reduced and catalyst particles having a low ratio of hollow particles and a high wear resistance strength and compressive strength are obtained. Meanwhile, when the solid concentration is reduced, the amount of catalyst produced tends to be reduced. Thus, the above solid concentration is preferably more than 3% by mass, and more preferably 10% by mass or more, from the viewpoint of the improvement in the shape of the resulting catalyst.

The upper limit of the solid concentration is more preferably 27% by mass, and further preferably 24% by mass or less.

In this regard, the solid concentration is represented by (mass of solid component in precursor slurry) $\times$ 100 / (mass of precursor slurry). The solid component refers to a portion obtained by excluding crystal water from a solid, the solid being added when preparing a precursor slurry. For example, liquids such as water, nitric acid, ammonia water and aqueous hydrogen peroxide, which are added to a precursor slurry, are not included in the solid component. Furthermore, in the case of solids containing crystal water, such as ammonium molybdate tetrahydrate, oxalic acid dihydrate and metal nitrate, the portion of crystal water is not included in the solid component.

It is preferable that the starting material for the respective components used for preparing a precursor slurry is a salt soluble in water or nitric acid. The starting material for elements molybdenum, bismuth, and iron is not particularly limited, and examples thereof include ammonium salts, nitrates, hydrochlorides, sulfates, organic acid salts, and inorganic salts which are soluble in water or nitric acid. In particular, ammonium salts are preferred as the starting material for molybdenum. Furthermore, as the starting material for bismuth and the starting material for iron, nitrates of each are preferred. Nitrates are also preferred in that they are easy to handle and that chlorine does not remain unlike in the case of using hydrochloride, or sulfur does not remain unlike in the case of using sulfate. Examples of starting materials for each component include ammonium paramolybdate, bismuth nitrate and ferric nitrate.

The temperature of the precursor slurry is preferably 20 to 60°C.

Silica sol is preferred as the starting material for silica. A preferred concentration of silica sol in the form of a starting material to which no other metal components are mixed is 10 to 50% by mass.

A carboxylic acid compound may be added to the precursor slurry where necessary. Carboxylic acids are a typical organic coordination compound, and are considered to facilitate high dispersibility of metal components by bonding to the metal components. Carboxylic acid compounds are not particularly limited, and two or higher valent polycarboxylic acids are preferred. Examples thereof include oxalic acid, tartaric acid, succinic acid, malic acid, and citric acid. Of them, oxalic acid and tartaric acid are preferred, and oxalic acid is more preferred.

[Step (ii): Step of drying]

Step (ii) is a step of spray-drying the precursor slurry to thereby obtain the dried particle. Spherical fine particles suitable for fluidized bed reaction can be obtained by spray-drying the precursor slurry. The system of the spray-drying apparatus used in this step is not particularly limited, and methods which are industrially typically used, such as a centrifugal system, a two-fluid nozzle system, and a high-pressure nozzle system may be used. The particle diameter of the catalyst may be adjusted by arranging conditions of spray-drying. When used as a fluidized bed catalyst, catalyst particles have an average particle diameter of preferably 25 to 180 $\mu$m.

The temperature of air at the inlet of the spray-drying apparatus is preferably 120 to 240°C, and more preferably 150 to 220°C, from the viewpoint of adjusting the temperature of the surface of droplets sprayed to thereby reduce the ratio of hollow particles.

The form of the spray-drying apparatus is not particularly limited. The inner diameter of the spray-drying apparatus is represented by the maximum diameter of the drying chamber portion. Usually a precursor slurry is sprayed from the center of the upper portion of the drying chamber of a spray-drying apparatus, and is dried by contacting heated air separately supplied from the upper portion of the drying chamber with the precursor slurry sprayed. The dried particles obtained in this way are taken out from the lower portion of the drying chamber of the spray-drying apparatus and supplied to the subsequent step (step of calcination).

The catalyst particles produced by the method of production of the present embodiment have a low ratio of hollow particles and exhibit high wear resistance strength and compressive strength. The present inventers consider this as follows.

It is assumed that the surface of droplets sprayed is rapidly dried in spray-drying of a precursor slurry to form an outer shell through which vapor is difficult to pass, and then when the wet portion inside the droplets sprayed is evaporated, particles are swelled to form hollow particles. A reduction of the solid concentration of the precursor slurry is considered to increase the amount of liquid components to be evaporated in spray-drying to reduce the temperature of the surface of droplets due to evaporation heat; and then particles are gradually dried without forming an outer shell to form solid particles.

Furthermore, when a large spray-drying apparatus is used, the ratio of hollow particles is increased compared to the case where a small spray-drying apparatus is used. This may be because since the amount of hot air is increased due to the enlargement of the apparatus, heat given to the precursor slurry is increased to increase the temperature on the surface of droplets sprayed. However, as shown in Examples described later, dried particles having a low ratio of hollow particles can be obtained in the present embodiment even when using a large apparatus with an inner diameter of a spray-drying apparatus of 1,000 mm or more, by controlling the above solid concentration in step (i) and the temperature of air at the inlet of a spray-drying apparatus in step (ii). Thus, it can be said that the method of the present embodiment is particularly suitable for use with a spray-drying apparatus having an inner diameter of 1,000 mm or more, more preferably 1,600 mm or more, and further preferably 2,000 mm or more. In short, catalyst particles having appropriate ratio of hollow particles are likely to be produced with higher efficiency by using a spray-drying apparatus having an inner diameter of 1,000 mm or more. In particular, the method of production of the present embodiment may also be applied to a spray-drying apparatus having an inner diameter of 5,000 mm or more, enabling production of a catalyst with even higher efficiency.

[Step (iii): Step of calcination]

Step (iii) is a step of calcining the dried particle obtained by spray-drying.

Particles are heat-treated and calcined by using a usual tunnel or rotary kiln. Calcination is performed in the range of the calcination temperature of preferably 500 to 750°C, and more preferably 500 to 680°C. The calcination time may vary depending on the calcination temperature, and is selected from the range of preferably 1 to 20 hours.

[Method for producing acrylonitrile]

The method for producing acrylonitrile of the present embodiment comprises the reaction step in which propylene, molecular oxygen and ammonia are reacted in the presence of the catalyst for ammoxidation described above to produce acrylonitrile.

Production of acrylonitrile by ammoxidation reaction may be performed by using a fixed bed reactor or a fluidized bed reactor. Of them, a fluidized bed reactor is preferred, from the viewpoint of efficiently removing heat generated during reaction and increasing the yield of acrylonitrile.

Propylene and ammonia which are starting materials in the ammoxidation reaction are not necessarily of high purity, and propylene and ammonia of industrial grade can be used. The molar ratio of propylene, ammonia, and oxygen (propylene/ammonia/oxygen) in the starting material gas is preferably 1.0 / 1.0 to 1.5 / 1.6 to 2.2.

The reaction temperature is preferably 380 to 480°C. Furthermore, the reaction pressure is preferably normal pressure to 0.3 MPa. The contact time of the starting material gas with the catalyst for ammoxidation is preferably 2 to 7 seconds, and more preferably 3 to 6 seconds.

Examples

Hereinafter, the present embodiment will be described in more detail with reference to Examples, but the present embodiment is not limited to Examples described below. The values of the composition of the catalysts shown in Examples and Comparative Examples are the same as the values of the composition of the elements added.

[Example 1]

Catalyst particles in which 60% by mass of a composite metal oxide having a metal composition represented by $Mo_{12.00}Bi_{0.37}Fe_{1.42}Co_{4.47}Ni_{3.30}Ce_{0.91}Rb_{0.14}$ was supported by 40% by mass of a carrier made of silica were produced according to the following procedure.

In a 650 liter container equipped with a stirrer, 2.85 kg of oxalic acid dihydrate dissolved in 32.81 kg of water was added to 152.17 kg of silica sol containing 30% by mass of $SiO_2$. 54.69 kg of ammonium paramolybdate $[(NH_4)_6Mo_7O_{24}\cdot4H_2O]$ dissolved in 107.73 kg of water was added thereto with stirring to prepare the first solution containing molybdenum and silica. The temperature of the solution was adjusted to 45°C.

Next, 4.70 kg of bismuth nitrate $[Bi(NO_3)_3\cdot5H_2O]$, 14.71 kg of iron nitrate $[Fe(NO_3)_3\cdot9H_2O]$, 33.90 kg of cobalt nitrate $[Co(NO_3)_2\cdot6H_2O]$, 24.97 kg of nickel nitrate $[Ni(NO_3)_2\cdot6H_2O]$, 10.14 kg of cerium nitrate $[Ce(NO_3)_3\cdot6H_2O]$ and 0.54 kg of rubidium nitrate $[RbNO_3]$ were dissolved in 56.99 kg of 16.6% by mass nitric acid to prepare the second solution. The temperature of the solution was adjusted to 40°C.

The first solution was mixed with the second solution to prepare a precursor slurry.

At that stage, the solid concentration of the precursor slurry was 30.0% by mass.

The resulting precursor slurry was dried by using a rotary disk type spray drier having an inner diameter of the drier of 5,400 mm. Here, the temperature of air at the inlet of the drier was set to 220°C. Furthermore, the number of revolutions of the disk was set to 7,000 revolutions/ minute.

The resulting dried particles were calcined at 540°C for 8 hours to produce a catalyst.

[Example 2]

A catalyst in which 60% by mass of a composite metal oxide represented by the same composition as that in Example 1 was supported by 40% by mass of a carrier made of silica was produced according to the following procedure.

In a 650 liter container equipped with a stirrer, 2.68 kg of oxalic acid dihydrate dissolved in 30.76 kg of water was added to 142.67 kg of silica sol containing 30% by mass of $SiO_2$. 51.28 kg of ammonium paramolybdate $[(NH_4)_6Mo_7O_{24}\cdot4H_2O]$ dissolved in 132.00 kg of water was added thereto with stirring to prepare the first solution containing molybdenum and silica. The temperature of the solution was adjusted to 45°C.

Next, 4.41 kg of bismuth nitrate $[Bi(NO_3)_3\cdot5H_2O]$, 13.80 kg of iron nitrate $[Fe(NO_3)_3\cdot9H_2O]$, 31.78 kg of cobalt nitrate $[Co(NO_3)_2\cdot6H_2O]$, 23.41 g of nickel nitrate $[Ni(NO_3)_2\cdot6H_2O]$, 9.51 kg of cerium nitrate $[Ce(NO_3)_3\cdot6H_2O]$ and 0.51 kg of rubidium nitrate $[RbNO_3]$ were dissolved in 53.43 kg of 16.6% by mass nitric acid to prepare the second solution. The temperature of the solution was adjusted to 40°C.

The first solution was mixed with the second solution to prepare a precursor slurry.

At that stage, the solid concentration of the precursor slurry was 28.1% by mass.

The subsequent steps were carried out in the same manner as in Example 1 to produce a catalyst.

[Example 3]

A catalyst was produced in the same manner as in Example 2 except for setting the drier inlet temperature to 210°C when drying the catalyst precursor slurry.

[Example 4]

A catalyst in which 60% by mass of a composite metal oxide represented by the same composition as that in Example 1 was supported by 40% by mass of a carrier made of silica was produced according to the following procedure.

In a 650 liter container equipped with a stirrer, 1.90 kg of oxalic acid dihydrate dissolved in 21.86 kg of water was added to 101.39 kg of silica sol containing 30% by mass of $SiO_2$. 36.44 kg of ammonium paramolybdate $[(NH_4)_6Mo_7O_{24} \cdot 4H_2O]$ dissolved in 237.37 kg of water was added thereto with stirring to prepare the first solution containing molybdenum and silica.

Next, 3.13 kg of bismuth nitrate $[Bi(NO_3)_3 \cdot 5H_2O]$, 9.80 kg of iron nitrate $[Fe(NO_3)_3 \cdot 9H_2O]$, 22.59 kg of cobalt nitrate $[Co(NO_3)_2 \cdot 6H_2O]$, 16.63 kg of nickel nitrate $[Ni(NO_3)_2 \cdot 6H_2O]$, 6.76 kg of cerium nitrate $[Ce(NO_3)_3 \cdot 6H_2O]$ and 0.36 kg of rubidium nitrate $[RbNO_3]$ were dissolved in 37.97 kg of 16.6% by mass nitric acid to prepare the second solution. The temperature of the solution was adjusted to 40°C.

The first solution was mixed with the second solution to prepare a precursor slurry.

At that stage, the solid concentration of the precursor slurry was 20.0% by mass.

The subsequent steps were carried out in the same manner as in Example 3 to produce a catalyst.

[Example 5]

A catalyst in which 60% by mass of a composite metal oxide represented by the same composition as that in Example 1 was supported by 40% by mass of a carrier made of silica was produced according to the following procedure.

In a 650 liter container equipped with a stirrer, 0.95 kg of oxalic acid dihydrate dissolved in 10.91 kg of water was added to 50.60 kg of silica sol containing 30% by mass of $SiO_2$. 18.18 kg of ammonium paramolybdate $[(NH_4)_6Mo_7O_{24} \cdot 4H_2O]$ dissolved in 367.05 kg of water was added thereto with stirring to prepare the first solution containing molybdenum and silica.

Next, 1.56 kg of bismuth nitrate $[Bi(NO_3)_3 \cdot 5H_2O]$, 4.89 kg of iron nitrate $[Fe(NO_3)_3 \cdot 9H_2O]$, 11.27 kg of cobalt nitrate $[Co(NO_3)_2 \cdot 6H_2O]$, 8.30 kg of nickel nitrate $[Ni(NO_3)_2 \cdot 6H_2O]$, 3.37 kg of cerium nitrate $[Ce (NO_3)_3 \cdot 6H_2O]$ and 0.18 kg of rubidium nitrate $[RbNO_3]$ were dissolved in 18.95 kg of 16.6% by mass nitric acid to prepare the second solution. The temperature of the solution was adjusted to 40°C.

The first solution was mixed with the second solution to prepare a precursor slurry.

At that stage, the solid concentration of the precursor slurry was 10.0% by mass.

The subsequent steps were carried out in the same manner as in Example 3 to produce a catalyst.

[Example 6]

A catalyst in which 60% by mass of a composite metal oxide represented by the same composition as that in Example 1 was supported by 40% by mass of a carrier made of silica was produced according to the following procedure.

In a 650 liter container equipped with a stirrer, 0.29 kg of oxalic acid dihydrate dissolved in 3.29 kg of water was added to 15.24 kg of silica sol containing 30% by mass of $SiO_2$. 5.48 kg of ammonium paramolybdate $[(NH_4)_6Mo_7O_{24} \cdot 4H_2O]$ dissolved in 457.31 kg of water was added thereto with stirring to prepare the first solution containing molybdenum and silica.

Next, 0.47 kg of bismuth nitrate $[Bi(NO_3)_3 \cdot 5H_2O]$, 1.47 kg of iron nitrate $[Fe(NO_3)_3 \cdot 9H_2O]$, 3.40 kg of cobalt nitrate $[Co(NO_3)_2 \cdot 6H_2O]$, 2.50 kg of nickel nitrate $[Ni(NO_3)_2 \cdot 6H_2O]$, 1.02 kg of cerium nitrate $[Ce (NO_3)_3 \cdot 6H_2O]$ and 0.05 kg of rubidium nitrate $[RbNO_3]$ were dissolved in 5.71 kg of 16.6% by mass nitric acid to prepare the second solution. The temperature of the solution was adjusted to 40°C.

The first solution was mixed with the second solution to prepare a precursor slurry.

At that stage, the solid concentration of the precursor slurry was 3.0 % by mass.

The subsequent steps were carried out in the same manner as in Example 1 except for setting the drier inlet temperature to 120°C when drying the catalyst precursor slurry to produce a catalyst.

The amount of catalyst obtained by the above procedure was small, and the result was a reduction in productivity.

[Example 7]

A catalyst was produced in the same manner as in Example 3 except for using a drier having an inner diameter the drier of 1,600 mm when drying the catalyst precursor slurry.

[Comparative Example 1]

A catalyst in which 60% by mass of a composite metal oxide represented by the same composition as that in Example 1 was supported by 40% by mass of a carrier made of silica was produced according to the following procedure.

In a 650 liter container equipped with a stirrer, 2.97 kg of oxalic acid dihydrate dissolved in 34.13 kg of water was added to 158.30 kg of silica sol containing 30% by mass of $SiO_2$. 56.89 kg of ammonium paramolybdate $[(NH_4)_6Mo_7O_{24}\cdot 4H_2O]$ dissolved in 92.09 kg of water was added thereto with stirring to prepare the first solution containing molybdenum and silica.

Next, 4.89 kg of bismuth nitrate $[Bi(NO_3)_3\cdot 5H_2O]$, 15.31 kg of iron nitrate $[Fe(NO_3)_3\cdot 9H_2O]$, 35.27 kg of cobalt nitrate $[Co(NO_3)_2\cdot 6H_2O]$, 25.97 kg of nickel nitrate $[Ni(NO_3)_2\cdot 6H_2O]$, 10.55 kg of cerium nitrate $[Ce(NO_3)_3\cdot 6H_2O]$ and 0.56 kg of rubidium nitrate $[RbNO_3]$ were dissolved in 59.28 kg of 16.6% by mass nitric acid to prepare the second solution. The temperature of the solution was adjusted to 40°C.

The first solution was mixed with the second solution to prepare a precursor slurry.

At that stage, the solid concentration of the precursor slurry was 31.2% by mass.

The subsequent steps were carried out in the same manner as in Example 3 to produce a catalyst.

[Comparative Example 2]

A catalyst was produced in the same manner as in Example 2 except for setting the drier inlet temperature to 250°C when drying the catalyst precursor slurry.

[Evaluation of ratio of hollow particles]

Calcined catalyst particles were embedded in an epoxy resin. Next, the resin in which the particles were embedded was polished to expose a cross-section of catalyst particles. Subsequently, osmium was deposited on the polished resin sample and the polished cross-section was observed by an electron microscope (Scanning Electron Microscope S-4800 made by Hitachi High-Technologies Corporation). The cross-section was photographed at a magnification of 100. Images were taken until the total number of particles reached 1,500 particles or more. Here, a particle in which the total area occupied by voids was 1% or more based on the area of a cross-section of a particle was determined as a hollow particle. The total number of particles and the number of hollow particles in the photographed images were counted, and the ratio of hollow particles was calculated by dividing the number of hollow particles by the total number of particles and multiplying the resultant by 100.

[Measurement of wear resistance strength]

The wear resistance strength (attrition strength) of the catalysts was measured in terms of wear loss according to the method described in the "Test Method for Synthetic Fluid Cracking Catalyst" (American Cyanamid Co. Ltd. 6/31-4m-1/57) (hereinafter referred to as the "ACC method").

The attrition strength is assessed based on wear loss. The wear loss is defined as follows.

$$\text{Wear loss (\%)} = R\ /\ (S-Q)\ \times\ 100$$

In the above formula, Q represents the mass (g) of the catalyst which was worn and scattered to the outside during the period from 0 to 5 hours, and R usually represents the mass (g) of the catalyst which was worn and scattered to the outside during the period from 5 to 20 hours. S represents the mass (g) of the catalyst supplied to the test.

Those with a value of wear loss of 3% or less were evaluated as applicable to industrial use. Those with a value of wear loss of 0.8% or less were evaluated as being able to be used for long time in a stable manner.

[Measurement of compressive strength]

The compressive strength of catalyst particles was measured by using Micro Compression Tester (MCT-W500 made by Shimadzu Corporation). For catalyst particles whose particle diameter was previously measured by a microscope attached to the tester, the data of compressive strength (MPa) was collected by using a 200 $\mu$m diameter flat cylindrical indenter at a rate of 19.4 mN/sec.

The compressive strength is calculated by the following formula.

$$\text{Compressive strength (MPa)} = a\ \times\ P/\pi\ \times\ d\ \times\ d$$

In the above formula, a represents the constant 2.48 in accordance with JISR1639-5, P represents the load (N) at breakdown and d represents the particle diameter (mm).

20 particles were measured by the above procedure and the arithmetic mean of the values obtained were calculated and determined as the value of compressive strength.

[Conditions of Ammoxidation Reaction and Results]

A Pyrex (registered trademark) glass pipe having an inner diameter of 25 mm and containing 16 10-mesh wire nets at intervals of 1 cm was used as a reaction pipe to be used for ammoxidation reaction of propylene. The amount of catalyst was set to 50 cc, the reaction temperature was set to 430°C and the reaction pressure was set to 0.17 MPa. A mixed gas (propylene, ammonia, oxygen, helium) with 9% by volume of propylene was passed through the pipe. The volume ratio of ammonia to propylene was set so that the sulfuric acid unit requirement defined by the following equation was 20 ± 2 kg/T-AN. The volume ratio of oxygen to propylene was set so that the oxygen concentration of gas at the outlet of the reactor was 0.2 ± 0.02% by volume. The contact time defined by the following equation can be changed by changing the flow rate of the mixed gas. Accordingly, the contact time was set so that the conversion rate of propylene defined by the following equation was 99.3 ± 0.2%. The yield of acrylonitrile produced by the reaction is defined as shown in the following formula.

$$\text{Sulfuric acid unit requirement (kg/T-AN)} = \frac{\text{Weight of sulfuric acid needed to neutralize unreacted ammonia (kg)}}{\text{Weight of acrylonitrile produced (T)}}$$

$$\text{Contact time (sec.)} = \frac{\text{Amount of catalyst (cc)}}{\text{Flow rate of mixed gas (cc-NTP/sec.)}} \times \frac{273}{273 + \text{Reaction temperature (°C)}} \times \frac{\text{Reaction pressure (MPa)}}{0.10}$$

$$\text{Conversion rate of propylene (\%)} = \frac{\text{Propylene consumed (mol)}}{\text{Propylene supplied (mol)}} \times 100$$

$$\text{Yield of acrylonitrile (\%)} = \frac{\text{Acrylonitrile produced (mol)}}{\text{Propylene supplied (mol)}} \times 100$$

The solid concentration of the slurries of catalyst precursor obtained in Examples and Comparative Examples, the drier inlet temperature, the inner diameter of the drier, the ratio of hollow particles, the compressive strength, the wear resistance strength and the yield of acrylonitrile are shown in Table 1. The reaction time was 20 hours.

[Table 1]

|  | Solid concentration (%) | Drier inlet temperature (°C) | Inner diameter of drier (mm) | Ratio of hollow particles (%) | Compressive strength (Mpa) | Wear resistance strength (%) | Yield of acrylonitrile (%) |
|---|---|---|---|---|---|---|---|
| Example 1 | 30.0 | 220 | 5400 | 23 | 38 | 0.8 | 84.4 |
| Example 2 | 28.1 | 220 | 5400 | 16 | 40 | 0.7 | 84.5 |
| Example 3 | 28.1 | 210 | 5400 | 9 | 42 | 0.6 | 84.4 |
| Example 4 | 20.0 | 210 | 5400 | 7 | 42 | 0.6 | 84.4 |
| Example 5 | 10.0 | 210 | 5400 | 5 | 42 | 0.6 | 84.4 |
| Example 6 | 3.0 | 120 | 5400 | 0 | 47 | 0.2 | 84.3 |
| Example 7 | 28.1 | 210 | 1600 | 1 | 46 | 0.2 | 84.4 |
| Comparative Example 1 | 31.2 | 210 | 5400 | 24 | 29 | 0.9 | 84.3 |
| Comparative Example 2 | 28.1 | 250 | 5400 | 38 | 30 | 1.3 | 84.4 |

As shown in Table 1 above, the ratio of hollow particles and the wear resistance strength of the catalysts produced according to the present embodiment were low, and it is indicated that the strength of catalyst was increased.
Furthermore, acrylonitrile was produced at an excellent yield by ammoxidation reaction of propylene.
The present application claims the priority based on Japanese Patent Application filed on May 19, 2017 (Japanese Patent Application No. 2017-100007), the contents of which are hereby incorporated by reference.

Industrial Applicability

The catalyst for ammoxidation of the present invention has industrial applicability as a catalyst used for ammoxidation reaction of propylene.

**Claims**

1. A catalyst for ammoxidation, comprising a catalyst particle comprising molybdenum, bismuth and iron, wherein the catalyst has a ratio of hollow particles of 23% or less.

2. The catalyst for ammoxidation according to claim 1, wherein the catalyst particle comprises a composite metal oxide having a composition represented by the following formula (1):

$$Mo_{12}Bi_aFe_bX_cY_dZ_eO_f \qquad (1)$$

wherein X represents at least one element selected from the group consisting of nickel, cobalt, magnesium, calcium, zinc, strontium, and barium; Y represents at least one element selected from the group consisting of cerium, chromium, lanthanum, neodymium, yttrium, praseodymium, samarium, aluminum, gallium, and indium; Z represents at least one element selected from the group consisting of potassium, rubidium, and cesium; and a, b, c, d, e, and f represent an atomic ratio of each element and satisfy $0.1 \leq a \leq 2.0$, $0.1 \leq b \leq 3.0$, $0.1 \leq c \leq 10.0$, $0.1 \leq d \leq 3.0$, and $0.01 \leq e \leq 2.0$, respectively, wherein f represents a number of oxygen atoms needed to satisfy atomic valence requirements of the other existing elements.

3. A method for producing the catalyst for ammoxidation according to claim 1 or 2, the method comprising:

   a step of preparing a catalyst precursor slurry which comprises molybdenum, bismuth and iron and which has a solid concentration of 30% by mass or less,
   a step of spray-drying the catalyst precursor slurry at a drier inlet temperature of 120°C to 240°C to thereby obtain a dried particle, and
   a step of calcining the dried particle at 500 to 750°C.

4. The method for producing the catalyst for ammoxidation according to claim 3, wherein the catalyst precursor slurry has a solid concentration of more than 3% by mass and 30% by mass or less.

5. The method for producing the catalyst for ammoxidation according to claim 3 or 4, wherein a spray-drying apparatus having an inner diameter of 1,000 mm or more is used.

6. A method for producing acrylonitrile, the method comprising a step of reacting propylene, molecular oxygen and ammonia in a presence of the catalyst for ammoxidation according to claim 1 or 2.

<table>
<tr><td colspan="2" align="center">**INTERNATIONAL SEARCH REPORT**</td><td colspan="2">International application No.<br>PCT/JP2018/014907</td></tr>
<tr><td colspan="4">A. CLASSIFICATION OF SUBJECT MATTER<br>Int.Cl.  B01J23/887(2006.01)i, B01J37/00(2006.01)i, C07C253/26(2006.01)i,<br>       C07C255/08(2006.01)i, C07B61/00(2006.01)n<br><br>According to International Patent Classification (IPC) or to both national classification and IPC</td></tr>
</table>

B. FIELDS SEARCHED

Minimum documentation searched (classification system followed by classification symbols)
Int.Cl. B01J23/887, B01J37/00, C07C253/26, C07C255/08, C07B61/00

| Documentation searched other than minimum documentation to the extent that such documents are included in the fields searched | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 57-171437 A (EUTECO IMPIANTI S.P.A) 22 October 1982, claims, page 3, lower left column, line 20 to lower right column, line 9, examples 3, 4, 7, 8, 10 & US 4388223 A, column 1, lines 24-33, examples 3, 4, 7, 8, 10, claims & DE 3114709 A1 & FR 2502977 A1 | 1-6 |

☒ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    22 June 2018 (22.06.2018) | Date of mailing of the international search report<br>    03 July 2018 (03.07.2018) |
|---|---|
| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2018/014907

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2004-313992 A (DAIYANITORIKKUSU KK) 11 November 2004, claims, examples 1, 2 & US 2006/0194693 A1:claims, examples 1, 2 & WO 2004/091776 A1 & EP 1634645 A1 & KR 10-2005-0120802 A & CN 1774294 A & BR PI0409407 A & KR 10-1043880 B1 | 1-6 |
| A | WO 2017/013115 A1 (EVONIK DEGUSSA GMBH) 26 January 2017, page 8, lines 11-13, claims & EP 3120926 A1 & CN 107847922 A | 1-6 |
| A | US 2016/0279618 A1 (CLARIANT CORPORATION) 29 September 2016, example 1, claims & WO 2016/153760 A1 | 1-6 |
| A | JP 2006-223941 A (DAIYANITORIKKUSU KK) 31 August 2006, claims, examples 2, 7, comparative example 1, table 1 (Family: none) | 1-6 |
| A | JP 50-125984 A (ASAHI KASEI INDUSTRY CO., LTD.) 03 October 1975, claims, examples (Family: none) | 1-6 |
| A | JP 2005-246372 A (DAIYANITORIKKUSU KK) 15 September 2005, claims, examples (Family: none) | 1-6 |
| A | JP 2009-285581 A (ASAHI KASEI CHEMICALS CORP.) 10 December 2009, claims, examples (Family: none) | 1-6 |
| A | JP 2002-301373 A (MITSUBISHI CHEMICAL CORP.) 15 October 2002, claims, paragraph [0027] (Family: none) | 1-6 |
| A | JP 2016-120468 A (ASAHI KASEI CHEMICALS CORP.) 07 July 2016, entire text (Family: none) | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5491037 B
- WO 2010016338 A

- JP 2017100007 A